# FASCICULE DE BREVET EUROPEEN

(11) **EP 1 057 809 B1**
(45) Date de publication et mention de la délivrance du brevet: **23.10.2002**
(21) Numéro de dépôt: 00401268.8
(22) Date de dépôt: 05.05.2000
(51) Int. Cl.: C07C 403/12, A61K 31/21, A61K 31/575, A61K 7/48, C07J 9/00, C07J 1/00, C07J 71/00, A61P 17/10

(54) **Dérivés carbonates de rétinol, procédé de préparation et utilisations**
Karbonatderivate von Retinol, Verfahren zu deren Herstellung und Verwendungen
Carbonate derivatives of retinol, process for their preparation and utilisations

(30) Priorité: 01.06.1999 FR 9906872
(43) Date de publication de la demande: 06.12.2000
(73) Titulaire: L'OREAL, 75008 Paris (FR)
(72) Inventeur: Tuloup, Rémy, 75014 Paris (FR); Rubinstenn, Gilles, 75013 Paris (FR); Dalko, Maria, 91190 Gif S/Yvette (FR)
(74) Mandataire: Renard, Emmanuelle

(56) Documents cités:
- WO-A-95/16659

## Description

La présente invention concerne de nouveaux dérivés carbonates de rétinol. Elle concerne également l'utilisation de ces composés dans ou pour la préparation de compositions destinées, notamment, à prévenir ou lutter contre l'acné et/ou le vieillissement chronologique ou actinique de la peau.

Les compositions cosmétiques et/ou dermatologiques à base de rétinoïdes ont connu un important développement au cours de ces dernières années.

En effet, il est apparu que certains composés de la famille des rétinoïdes, dérivés de l'acide rétinoïque, présentaient un intérêt pour le soin de la peau, notamment dans le traitement de l'acné et pour limiter, voire supprimer, les effets du vieillissement sur la peau que sont les rides, l'aspect parcheminé, le jaunissement, la perte d'élasticité, la rugosité et la sécheresse de la peau, ainsi que l'apparition de tâches.

Ces signes de vieillissement sont d'autant plus accentués que la peau a été fréquemment exposée au soleil ou est particulièrement sensible à l'exposition aux rayonnements UV. Ainsi, les effets du vieillissement intrinsèque de la peau (lié à l'âge) et du photo-vieillissement (dû à l'exposition au soleil) peuvent se cumuler. Les manifestations du vieillissement apparaissent habituellement à un âge avancé ; toutefois, leur prévention doit être entreprise dès le début de la vie d'adulte par des soins appropriés.

Le traitement de la peau par des composés de la famille des rétinoïdes fait partie de ces soins préventifs ou curatifs des manifestations du vieillissement.

Parmi les composés de la famille des rétinoïdes, le rétinol, également connu sous le nom de vitamine A, présente un intérêt tout particulier. En effet, le rétinol est un constituant endogène naturel de l'organisme humain. Il est en outre bien toléré en application sur la peau jusqu'à des taux beaucoup plus élevés que l'acide rétinoïque.

Toutefois, lorsqu'il est introduit dans une composition cosmétique destinée à une application topique, le rétinol se dégrade rapidement, sous l'effet de la lumière, de l'oxygène, des ions métalliques, des agents oxydants, de l'eau ou en particulier sous l'effet d'une élévation de température.

Il reste donc le besoin de composés à activité de type rétinoïde qui soient plus stables que le rétinol.

On connaît de WO 95/16659 des composés conjugués issus de la réaction de rétinoïdes sur des acides organiques ou des dérivés de ces acides. Le rétinoïde, qui peut être le rétinol, le rétinal, l'acide rétinoïque ou le déhydrorétinol, est conjugué au dérivé d'acide, qui peut être un aldéhyde, une cétone, un alcool, un ester, etc. par une liaison ester, ester inverse, amide ou éther. Parmi le très grand nombre de composés répondant à cette définition, seul l'éther obtenu par réaction du rétinol sur la fonction hydroxyle de l'acide glycolique est décrit. En particulier, il n'est pas fait mention des dérivés carbonates du rétinol.

Par ailleurs, les publications de Svishchuk et al., Preparation of ∝-tocopherol (Vitamin E) Ethers, Farm. Zh., 29(6), 36-8 (1974) et de Protopova et al., Insecticidal Action of 6-hydroxychroman derivatives, Fiziol. Akt. Veshchestva, 7, 142-4 (1975) décrivent respectivement un carbonate de tocophéryle et de rétinyle, et un composé analogue dans lequel la chaîne latérale du radical tocophéryle est légèrement modifiée. Ces composés ne sont toutefois pas destinés à une application cosmétique.

Les dérivés de rétinol selon l'invention répondent à la formule (I) suivante : 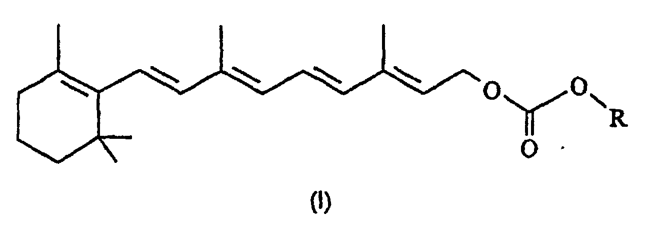 dans laquelle R est choisi dans le groupe constitué par :
(a) un radical hydrocarboné en C₁-C₁₈ linéaire, cyclique ou ramifié, saturé ou insaturé, non substitué ou substitué par un ou plusieurs groupements -OH, -OR', -NHCOR, -SH, -SR', -COOH, -CONHR', -COOR', -CN, -CF₃, où R' est un radical alkyle en C₁-C₄; par un atome d'halogène ; ou par :
   (i) un ou plusieurs cycles aliphatiques en C₃-C₇, saturés ou insaturés, substitués ou non substitués, ou
   (ii) un ou plusieurs cycles aromatiques, substitués ou non substitués, ou
   (iii) un ou plusieurs hétérocycles en C₃-C₇, saturés ou insaturés, substitués ou non substitués, ou
   (iv) un ou plusieurs restes d'aminoacide ou de peptide,
(b) un radical de structure cyclopentanephénanthrénique, au moins partiellement hydrogéné et/ou substitué,
(c) un radical aryle éventuellement substitué par un ou plusieurs groupements -OH, -NH₂, -SH, -COOH, -CONHR', -NHCOR, -COOR', -OR', -SR', -CN, -CF₃, où R' est un alkyle en C₁-C₄ ; par un atome d'halogène ; ou par
   (i) un ou plusieurs cycles aliphatiques en C₃-C₇, saturés ou insaturés, substitués ou non substitués, ou
   (il) un ou plusieurs cycles aromatiques, substitués ou non substitués, ou
   (iii) un ou plusieurs hétérocycles en C₃-C₇, saturés ou insaturés, substitués ou non substitués,
   étant entendu que les groupements chromanes substitués sont exclus
(d) un groupement dérivé de céramide, de sphingosine ou de sphinganine ;
(e) un reste d'aminoacide ou de peptide portant un groupe hydroxyle libre ; et
(f) un reste de carbohydrate.

Les dérivés de formule (I) peuvent être définis comme les produits de condensation du rétinol sur d'autres composés portant une fonction alcool, via une liaison carbonate.

Selon la présente invention, parmi les radicaux hydrocarbonés linéaires ayant de 1 à 18 atomes de carbone, on peut citer notamment les radicaux méthyle, éthyle, propyle, butyle, hexyle, octyle, décyle, dodécyle, hexadécyle et octadécyle.

Parmi les radicaux hydrocarbonés ramifiés ayant de 1 à 18 atomes de carbone, on peut citer notamment les radicaux 2-éthyl-hexyle, 2-butyl-octyle ou 2-hexyl-décyle.

Les radicaux hydrocarbonés insaturés sont de préférence des radicaux présentant une ou plusieurs insaturations éthyléniques, tels que les radicaux 2-nonyl 2-butényle, géranyle, 2,4-cyclohexadiényle et allyle.

Comme radicaux hydrocarbonés cycliques ayant de 1 à 18 atomes de carbone et cycles aliphatiques en C₃-C₇, on peut notamment citer les radicaux cyclobutyle, cyclohexyle et terbutylcyclohexyle.

Les radicaux hydrocarbonés linéaires, ramifiés ou cycliques en C₁-C₁₈ substitués par des groupements hydroxyle comprennent notamment les restes de polyols, tels que les radicaux 2,3-dihydroxypropyle, 2,3,4-trihydroxybutyle, 2,3,4,5-tétrahydroxypentyle et 2,3,4,5,6-pentahydroxyhexyle ou un reste de pentaérythritol, et de préférence les restes de sucres, dérivant par exemple du glucose, du galactose ou du mannose, ou bien encore de l'acide glucuronique.

Les radicaux hydrocarbonés linéaires, ramifiés ou cycliques en C₁-C₁₈ substitués par des groupements -COOH comprennent notamment les restes d'α-hydroxyacides, éventuellement sous forme lactonisée, ou de β-hydroxyacides, tels qu'un reste d'acide malique, tartrique, glycolique, lactique, ascorbique, salicylique ou de leurs dérivés.

Les radicaux de structure cyclopentanephénanthrénique au moins partiellement hydrogénés et/ou substitués comprennent notamment les radicaux stéryle et oxystéryle, et en particulier le radical cholestéryle ; les restes de sapogénines, et en particulier de diosgénine, d'hécogénine et de smilagénine et un reste de déhydro-épi-androstérone.

Par hétérocycle en C₃-C₇, on entend notamment la pyridine, le furanne, le thiophène et le pyrrole.

Par radical aryle et cycle aromatique, on entend de préférence un radical phényle éventuellement substitué par au moins un atome d'halogène, un hydroxyle ou une fonction nitro.

Par reste d'aminoacide, on entend notamment un reste dérivant de la lysine, de la glycine, de l'acide aspartique ou de la cystéine, et par reste de peptide on entend plus particulièrement un reste de dipeptide ou de tripeptide résultant de la combinaison d'acides aminés. Comme reste d'aminoacide portant un groupe hydroxyle libre, on peut notamment citer la sérine et la tyrosine.

Par reste de carbohydrate, on entend un reste dérivant notamment de glucose, de galactose, de mannose, de lactose, de mélibiose, de sorbitol, ou bien encore de l'acide glucuronique.

De préférence, le dérivé de rétinol selon l'invention est le carbonate de rétinyle et de cholestéryle, qui répond à la formule générale (I) ci-dessus dans laquelle R est un radical cholestéryle.

Selon une autre forme d'exécution de l'invention, le dérivé de rétinol de formule (I) est le carbonate de rétinyle, c'est-à-dire que R représente un radical rétinyle.

L'invention étend également sa portée à un procédé de préparation des dérivés carbonates de rétinol précités.

Selon une première voie de synthèse, qui permet de préparer l'ensemble des composés de formule (I), le procédé selon l'invention comprend l'étape consistant à faire réagir un chloroformiate sur le rétinol. La réaction est avantageusement effectuée en milieu anhydre et sous atmosphère inerte, de manière à protéger le rétinol vis-à-vis de l'oxydation qu'il a tendance à subir en milieu aqueux et/ou au contact de l'oxygène de l'air.

La réaction mise en oeuvre est illustrée à la figure (II) :

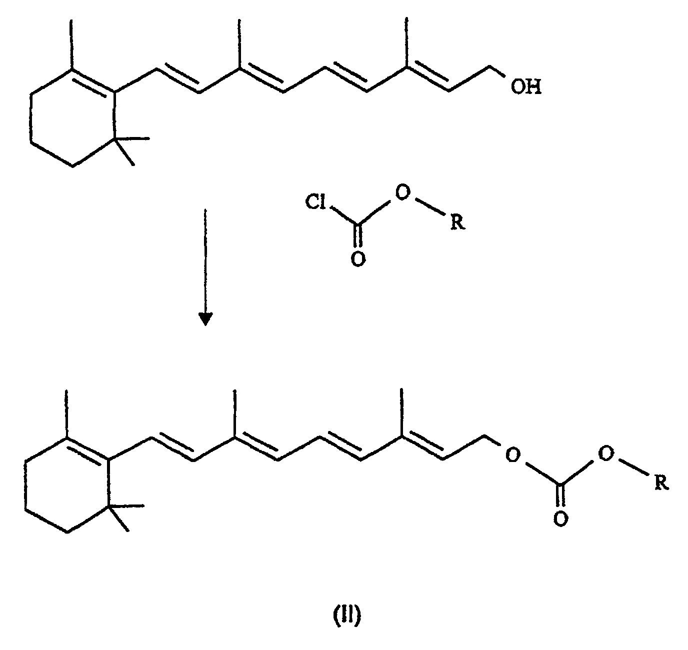

Selon une seconde voie de synthèse, qui peut être utilisée dans le cas où le dérivé carbonate de rétinol est le carbonate de rétinyle, le procédé selon l'invention comprend l'étape consistant à faire réagir deux équivalents de rétinol avec un équivalent de carbonyldiimidazole. Comme précédemment, la réaction est avantageusement effectuée en milieu anhydre et sous atmosphère inerte.

Le schéma réactionnel est illustré à la figure (III) :

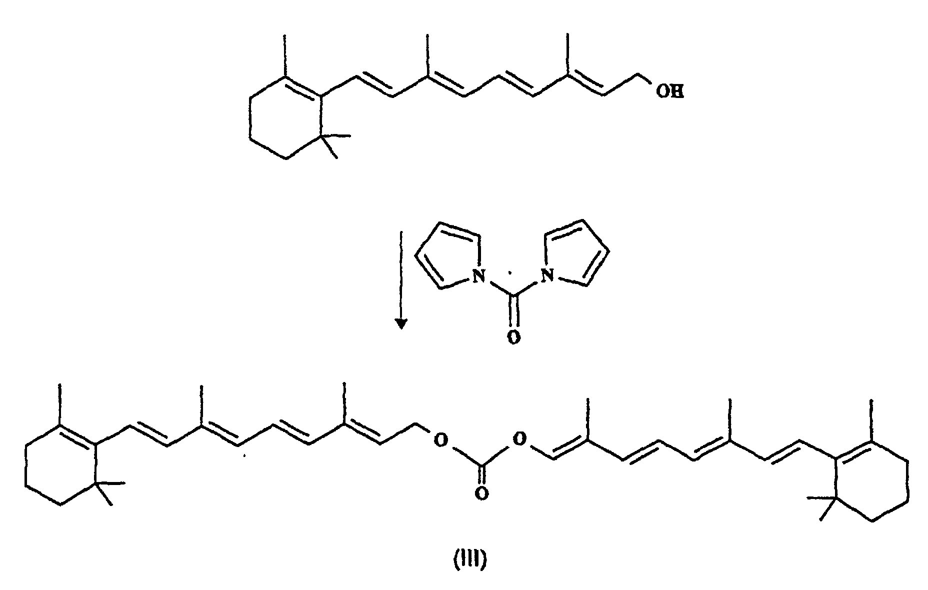

L'invention concerne également und composition comprenant, dans un milieu physiologiquement acceptable, au moins un dérivé carbonate de rétinol tel que défini ci-dessus. Par "physiologiquement acceptable", on entend un milieu compatible avec la peau et ses phanères (ongles, poils) et/ou avec les cheveux.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

La composition selon l'invention peut se présenter sous toutes les formes galéniques normalement utilisées pour une application topique sur la peau, notamment sous forme d'une solution aqueuse, hydroalcoolique ou huileuse, d'une émulsion huile-dans-eau ou eau-dans-huile ou multiple, d'une émulsion siliconée, d'une microémulsion ou nanoémulsion, d'un gel aqueux ou huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau, ou comme produit capillaire, par exemple comme shampooing ou après-shampooing.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants, ainsi que leurs concentrations, doivent être tels qu'ils ne nuisent pas aux propriétés avantageuses du dérivé carbonate de rétinol selon l'invention.

Lorsque la composition selon l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100 ; les esters d'acide gras et de glycérol tels que le stéarate de glycéryle ; les esters d'acide gras et de sorbitan, éventuellement oxyalkylénés ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs, on peut utiliser notamment les dépigmentants, les émollients, les hydratants, les anti-séborrhéiques, les anti-acnéiques, les agents favorisant la repousse des cheveux, les agents kératolytiques et/ou desquamants, les agents anti-irritants ou apaisants et leurs mélanges. Avantageusement, dans la composition selon l'invention, les dérivés carbonates de rétinol définis plus haut seront employés en combinaison avec d'autres composés à activité de type rétinoïde, avec des anti-radicaux libres, ou avec des α-hydroxy ou α-céto acides ou leurs dérivés. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou leurs sels, amides ou esters.

En cas d'incompatibilité, les actifs indiqués ci-dessus peuvent être incorporés dans des sphérules, notamment des vésicules ioniques ou non-ioniques et/ou des nanoparticules (nanocapsules et/ou nanosphères), de manière à les isoler les uns des autres dans la composition.

La composition selon l'invention trouve en particulier une application dans l'hygiène corporelle et capillaire et notamment pour le soin des peaux à tendance acnéique ou squameuses, pour lutter contre l'aspect gras de la peau ou des cheveux, pour lutter contre la chute des cheveux, dans la protection contre les aspects néfastes du soleil ou pour prévenir et/ou pour lutter contre le vieillissement actinique ou chronologique.

La présente invention concerne donc également l'utilisation de la composition mentionnée ci-dessus pour le traitement cosmétique de la peau, en particulier contre l'acné et/ou le vieillissement chronologique ou actinique. Elle concerne aussi l'utilisation de cette composition pour le traitement cosmétique du cuir chevelu, en particulier pour lutter contre l'aspect gras des cheveux et la chute des cheveux.

La présente invention concerne également l'utilisation des dérivés de rétinol définis ci-dessus pour préparer une composition destinée à lutter contre l'acné et/ou le vieillissement chronologique ou actinique de la peau. Elle concerne aussi l'utilisation de ces dérivés de rétinol pour préparer une composition destinée à lutter contre l'aspect gras des cheveux et la chute des cheveux.

Dans tous les cas, la composition selon l'invention comprend une quantité efficace d'au moins un dérivé carbonate de rétinol tel que défini ci-dessus, suffisante pour obtenir l'effet recherché, et un milieu physiologiquement acceptable. La composition selon l'invention renferme ainsi par exemple de 0,001 à 20% en poids, de préférence de 0,01 à 10% en poids, plus préférentiellement de 0,1 à 5% en poids de dérivé carbonate de rétinol, par rapport au poids total de la composition.

L'invention sera maintenant illustrée par les exemples non limitatifs suivants de procédés d'obtention des dérivés de rétinol de formule (I), ainsi que de formulations à base de ces composés.

### EXEMPLES

### Exemple 1 : carbonate de rétinyle et de cholestéryle

### a) Procédé de préparation

Un équivalent molaire de rétinol et un équivalent molaire de chloroformiate de cholestéryle sont dissous dans le dichlorométhane anhydre, sous atmosphère inerte, à température ambiante. De la pyridine (3,5 équivalents molaire) est ajoutée et le milieu réactionnel est maintenu sous agitation pendant 20 minutes.

Après dilution par du dichlorométhane, lavage à l'eau, concentration et co-évaporation de la pyridine par le toluène, le produit final de formule (IV) ci-dessous est recristallisé dans l'acétone.

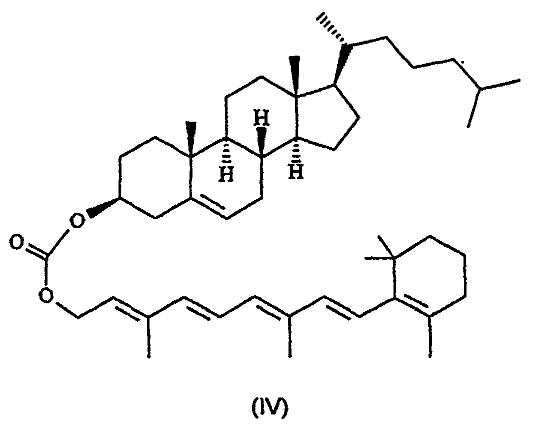

### b) Caractérisation par RMN

RMN ¹³C (100MHz dans CDCl₃) d (en ppm) : 154.82, 139.57, 139.42, 137.83, 137.57, 136.7, 135.72, 129.9, 125.35, 127.06, 126.02, 123.84, 122.89, 77.89, 64.26, 56.72, 56.18, 50.5, 42.34, 39.75, 39.66, 39.54, 38.07, 36.89, 36.56, 36.20, 35.78, 34.26, 33.06, 31.91, 31.87, 28.95 (2 C), 28.21, 28.00, 27.73, 24.28, 23.84, 22.79, 22.54, 21.69, 21.06, 19.27, 19.25, 18.72, 12.78, 12.72, 11.85.

RMN ¹H (400MHz dans CDCl₃) d (en ppm) : 6.64 (dd, 11.3Hz, 15.2Hz, 1H), 6.26 (d, 15.2Hz, 1H), 6.18 (d, 16.1Hz, 1H), 6.10 (d, 16.3Hz, 1H), 6.09 (d, 11.2Hz, 1H), 5.40-5.38 (m, 1H), 4.77 (d, 7.25, 2H), 4.51-4.44 (m, 1H), 2.40-0.85 (m, 62H), 0.67 (s, 3H).

### Exemple 2 : carbonate de rétinyle

### a) Procédé de préparation

On dissous deux équivalents de rétinol et un équivalent molaire de carbonyldiimidazole dans le tétrahydrofuranne anhydre, sous atmosphère inerte, à température ambiante. Le milieu réactionnel est soumis à une agitation, le tétrahydrofuranne étant porté à reflux, pendant deux heures.

Après concentration, reprise dans l'éther, lavage à l'eau et concentration, on obtient le produit de formule (V) ci-dessous avec une pureté supérieure à 90% (seules des traces de rétinol sont détectées par RMN ¹H).

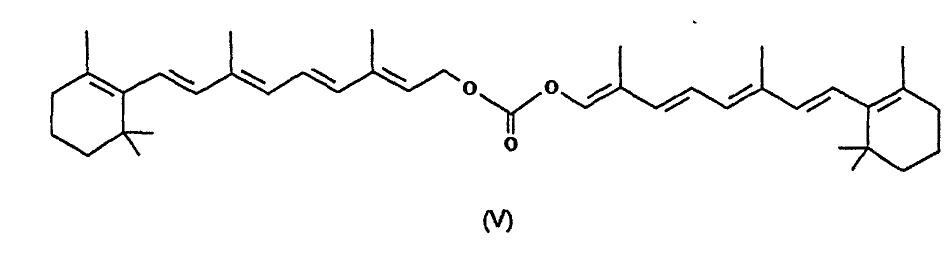

### b) Caractérisation par RMN

RMN ¹³C (50MHz dans CDCl₃) d (en ppm) : 155.2, 139.9, 137.9, 137.7, 136.9, 135.8, 130.0, 129.5, 127.2, 126.6, 123.8, 64.72, 37.3, 34.3, 33.2, 29.1, 21.9, 19.4, 12.9.

RMN ¹H (200MHz dans CDCl₃) d (en ppm) : 6.59 (dd, 11.2Hz, 15.0Hz, 2H), 6.20 (d, 15.0Hz, 2H), 6.10-5.97 (m, 4H), 5.60-5.52 (m, 2H), 4.72 (d, 7.26Hz, 4H), 1.97-1.36 (m, 32H), 0.95 (s, 12H).

### Exemple 3 :

Dans cet exemple, on a illustré diverses formulations cosmétiques à base des dérivés carbonates de rétinol selon l'invention.

| Crème anti-âge sous forme d'émulsion E/H | |
|---|---|
| | |
| Polyméthylcétyl diméthyl méthylsiloxane oxyéthyléné | 1,5% |
| Iso-stéarate polyglycérolé | 0,5% |
| Iso-hexadécane | 12,7% |
| Composé de l'Exemple 1 | 0,2% |
| Butylhydroxytoluène | 0,1% |
| Mélange de stéarate d'éthylène glycol acétylé et de tristéarate | |
| de glycéryle commercialisé par la société GUARDIAN | |
| sous la dénomination UNITWIX | 1 % |
| Conservateurs | 0,4% |
| Huile d'amandes d'abricot | 5 % |
| Glycérine | 5 % |
| Sulfate de magnésium | 0,7% |
| Sel disodique d'EDTA | 0,1% |
| lmidazolidinyl urée | 0,3% |
| Eau qsp. | 100 % |

| Fluide anti-âge sous forme d'émulsion H/E | |
|---|---|
| | |
| Mélange de mono-stéarate de glycéryle, stéarate de | |
| polyéthylèneglycol (100 OE) | 2,1% |
| Alcool cétylique | 2,6% |
| Iso-paraffine hydrogénée | 14,8% |
| Conservateurs | 0,4% |
| Composé de l'Exemple 1 | 0,1% |
| Butylhydroxytoluène | 0,1% |
| Mono-stéarate de sorbitane oxyéthyléné (20 OE) | 0,9% |
| Glycérine | 3 % |
| Gomme de xanthane | 0,1% |
| Carbomer | 0,4% |
| Imidazolidinyl urée | 0,3% |
| Triéthanolamine | 0,3% |
| Eau qsp. | 100 % |

| Fluide anti-âge sous forme d'émulsion H/E | |
|---|---|
| | |
| Tri-stéarate de sorbitane | 0,9% |
| Stéarate de polyéthylèneglycol (40 OE) | 2 % |
| Alcool cétylique | 4 % |
| Stéarate de glycéryle | 3 % |
| Composé de l'Exemple 2 | 0,4% |
| Butylhydroxytoluène | 0,1% |
| Isoparaffine hydrogénée | 1,8 % |
| Conservateurs | 0,40 % |
| Cyclopentasiloxane | 10 % |
| Imidazolidinyl urée | 0,3% |
| Eau qsp. | 100 % |

## Revendications

1. Dérivé carbonate de rétinol de formule (I) suivante : 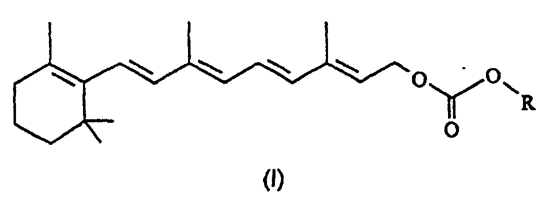 dans laquelle R est choisi dans le groupe constitué par
(a) un radical hydrocarboné en C₁-C₁₈ linéaire, cyclique ou ramifié, saturé ou insaturé, non substitué ou substitué par un ou plusieurs groupements -OH, -OR', -NHCOR, -SH, -SR', -COOH, -CONHR', -COOR', -CN, -CF₃, où R' est un radical alkyle en C₁-C₄ ; par un atome d'halogène ; ou par
(i) un ou plusieurs cycles aliphatiques en C₃-C₇, saturés ou insaturés, substitués ou non substitués, ou
(ii) un ou plusieurs cycles aromatiques, substitués ou non substitués,
(iii) un ou plusieurs hétérocycles en C₃-C₇, saturés ou insaturés, substitués ou non substitués, ou
(iv) un ou plusieurs restes d'aminoacide ou de peptide,
(b) un radical de structure cyclopentanephénanthrénique, au moins partiellement hydrogéné et/ou substitué,
(c) un radical aryle éventuellement substitué par un ou plusieurs groupements -OH, -NH₂, -SH, -COOH, -CONHR', -NHCOR, -COOR', -OR', -SR', -CN, -CF₃, où R' est un alkyle en C₁-C₄ ; par un atome d'halogène ; ou par :
(i) un ou plusieurs cycles aliphatiques en C₃-C₇, saturés ou insaturés, substitués ou non substitués, où
(ii) un ou plusieurs cycles aromatiques, substitués ou non substitués, ou
(iii) un ou plusieurs hétérocycles en C₃-C₇, saturés ou insaturés, substitués ou non substitués,
étant entendu que les groupements chromanes substitués sont exclus ;
(d) un groupement dérivé de céramide, de sphingosine ou de sphinganine ;
(e) un reste d'aminoacide ou de peptide portant un groupe hydroxyle libre ; et
(f) un reste de carbohydrate.

2. Dérivé de rétinol selon la revendication 1, **caractérisé en ce que** R représente un radical choisi parmi les radicaux stéryle et oxystéryle ; un reste de sapogénine ; et un reste de déhydro-épi-androstérone.

3. Dérivé de rétinol selon la revendication 2, **caractérisé en ce que** R représente un radical cholestéryle.

4. Dérivé de rétinol selon la revendication 1, **caractérisé en ce que** R représente un radical rétinyle.

5. Procédé de préparation du dérivé de rétinol selon l'une quelconque des revendications 1 à 4, comprenant l'étape consistant à faire réagir un chloroformiate sur le rétinol.

6. Procédé de préparation du dérivé de rétinol selon la revendication 4, comprenant l'étape consistant à faire réagir deux équivalents de rétinol avec un équivalent de carbonyldiimidazole.

7. Procédé selon la revendication 5 ou 6, **caractérisé en ce que** la réaction est effectuée en milieu anhydre et sous atmosphère inerte.

8. Composition comprenant, dans un milieu physiologiquement acceptable, au moins un dérivé de rétinol selon l'une quelconque des revendications 1 à 4.

9. Composition selon la revendication 8, **caractérisée en ce qu'**elle comprend en outre au moins un actif choisi parmi les dépigmentants, les émollients, les hydratants, les anti-séborrhéiques, les anti-acnéiques, les agents favorisant la repousse des cheveux, les agents kératolytiques et/ou desquamants, les agents anti-irritants ou apaisants et leurs mélanges.

10. Utilisation de la composition selon la revendication 8 ou 9 pour le traitement cosmétique de la peau, en particulier contre l'acné et/ou le vieillissement chronologique ou actinique.

11. Utilisation de la composition selon la revendication 8 ou 9 pour le traitement cosmétique du cuir chevelu, en particulier pour lutter contre l'aspect gras des cheveux et la chute des cheveux.

12. Utilisation du dérivé de rétinol selon l'une quelconque des revendications 1 à 4 pour préparer une composition destinée à lutter contre l'acné et/ou le vieillissement chronologique ou actinique de la peau.

13. Utilisation du dérivé de rétinol selon l'une quelconque des revendications 1 à 4 pour préparer une composition destinée à lutter contre l'aspect gras des cheveux et la chute des cheveux.

14. Procédé de traitement cosmétique de la peau, comprenant l'application sur la peau d'une composition selon la revendication 8 ou 9.

## Claims

1. Retinol carbonate derivative of formula (I) below: 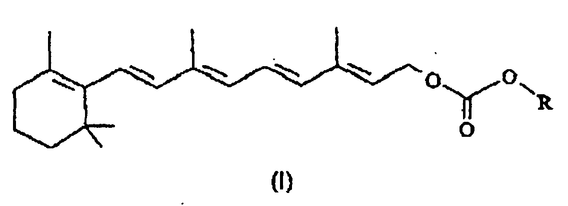 in which R is chosen from the group consisting of:
(a) a linear, cyclic or branched, saturated or unsaturated C₁-C₁₈ hydrocarbon-based radical, which is unsubstituted or substituted with one or more groups -OH, -OR', -NHCOR, -SH, -SR', -COOH, -CONHR', -COOR', -CN or -CF₃, in which R' is a C₁-C₄ alkyl radical; with a halogen atom; or with:
(i) one or more saturated or unsaturated, substituted or unsubstituted C₃-C₇ aliphatic rings, or
(ii) one or more substituted or unsubstituted aromatic rings, or
(iii) one or more saturated or unsaturated, substituted or unsubstituted C₃-C₇ heterocycles, or
(iv) one or more amino acid or peptide residues,
(b) a radical of cyclopentanephenanthrene structure, which is at least partially hydrogenated and/or substituted,
(c) an aryl radical optionally substituted with one or more groups -OH, -NH₂, -SH, -COOH, -CONHR', -NHCOR, -COOR', -OR', -SR', -CN or -CF₃, in which R' is a C₁-C₄ alkyl; with a halogen atom; or with:
(i) one or more saturated or unsaturated, substituted or unsubstitued C₃-C₇ aliphatic rings, or
(ii) one or more substituted or unsubstituted aromatic rings, or
(iii) one or more saturated or unsaturated, substituted or unsubstituted C₃-C₇ heterocycles, it being understood that substituted chroman groups are excluded;
(d) a group derived from ceramide, sphingosine or sphinganine;
(e) an amino acid or peptide residue bearing a free hydroxyl group; and
(f) a carbohydrate residue.

2. Retinol derivative according to Claim 1, **characterized in that** R represents a radical chosen from steryl and oxysteryl radicals; a sapogenin residue; and a dehydro-epi-androsterone residue.

3. Retinol derivative according to Claim 2, **characterized in that** R represents a cholesteryl radical.

4. Retinol derivative according to Claim 1, **characterized in that** R represents a retinyl radical.

5. Process for preparing the retinol derivative according to any one of Claims 1 to 4, comprising the step which consists in reacting a chloroformate with retinol.

6. Process for preparing the retinol derivative according to Claim 4, comprising the step which consists in reacting two equivalents of retinol with one equivalent of carbonyldiimidazole.

7. Process according to Claim 5 or 6, **characterized in that** the reaction is carried out in anhydrous medium and under an inert atmosphere.

8. Composition comprising, in a physiologically acceptable medium, at least one retinol derivative according to any one of Claims 1 to 4.

9. Composition according to Claim 8, **characterized in that** it also comprises at least one active agent chosen from depigmenting agents, emollients, moisturizers, anti-seborrhoeic agents, anti-acne agents, agents for promoting regrowth of the hair, keratolytic and/or desquamating agents, anti-irritant agents or calmants and mixtures thereof.

10. Use of the composition according to Claim 8 or 9 for the cosmetic treatment of the skin, in particular against acne and/or chronological or actinic ageing.

11. Use of the composition according to Claim 8 or 9 for the cosmetic treatment of the scalp, in particular to combat the greasy appearance of the hair and hair loss.

12. Use of the retinol derivative according to any one of Claims 1 to 4, to prepare a composition intended to combat acne and/or chronological or actinic ageing of the skin.

13. Use of the retinol derivative according to any one of Claims 1 to 4, to prepare a composition intended to combat the greasy appearance of the hair and hair loss.

14. Cosmetic treatment process for the skin, comprising the application of a composition according to Claim 8 or 9 to the skin.

## Patentansprüche

1. Carbonatderivat von Retinol der folgenden Formel (I); 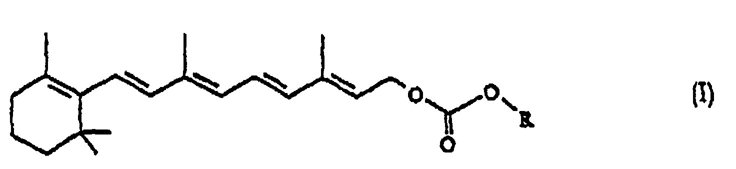 wobei R unter den folgenden Gruppen ausgewählt ist:
(a) einer geradkettigen, cyclischen oder verzweigten, gesättigten oder ungesättigten C₁₋₁₈-Kohlenwasserstoffgruppe, die unsubstituiert vorliegt oder mit einer oder mehreren Gruppen -OH, -OR', -NHCOR, -SH, -SR', -COOH, -CONHR', -COOR', -CN oder -CF₃, wobei R' eine C₁₋₄-Alkylgruppe ist; mit einem Halogenatom oder mit:
(i) einem oder mehreren gesättigten oder ungesättigten, substituierten und unsubstituierten aliphatischen C₃₋₇-Ringen, oder
(ii) einem oder mehreren substituierten oder unsubstituierten aromatischen Ringen oder
(iii) einem oder mehreren gesättigten oder ungesättigten, substituierten oder unsubstituierten C₃₋₇-Heterocyclen oder
(iv) einem oder mehreren Aminosäureresten oder Peptidresten,
substituiert ist;
(b) einer Gruppe mit Cyclopentanphenanthrenstruktur, die zumindest teilweise hydriert und/oder substituiert ist,
(c) einer Arylgruppe, die gegebenenfalls mit einer oder mehreren Gruppen -OH, -NH₂, -SH, -COOH, -CONHR', -NHCOR, -COOR', -OR', -SR', -CN oder -CF₃, wobei R' eine C₁₋₄-Alkylgruppe ist; mit einem Halogenatom oder mit:
(i) einem oder mehreren gesättigten oder ungesättigten, substituierten und unsubstituierten aliphatischen C₃₋₇-Ringen, oder
(ii) einem oder mehreren substituierten oder unsubstituierten aromatischen Ringen oder
(iii) einem oder mehreren gesättigten oder ungesättigten, substituierten oder unsubstituierten C₃₋₇-Heterocyclen substituiert ist,
wobei substituierte Chromangruppen ausgenommen sind;
(d) einer von Ceramid, Sphingosin oder Sphinganin abgeleiteten Gruppe;
(e) einem Aminosäurerest oder Peptidrest mit freier Hydroxygruppe; und
(f) einem Kohlenhydratrest.

2. Retinolderivat nach Anspruch 1, **dadurch gekennzeichnet, daß** die Gruppe R unter den Gruppen Steryl und Oxysteryl; einem Sapogeninrest; und dem Dehydroepiandrosteronrest ausgewählt ist.

3. Retinolderivat nach Anspruch 2, **dadurch gekennzeichnet, daß** R die Cholesterylgruppe ist.

4. Retinolderivat nach Anspruch 1, **dadurch gekennzeichnet, daß** R die Retinylgruppe ist.

5. Verfahren zur Herstellung eines Retinolderivats nach einem der Ansprüche 1 bis 4, das den Schritt der Umsetzung eines Chlorformiats mit Retinol umfaßt.

6. Verfahren zur Herstellung eines Retinolderivats nach Anspruch 4, das den Schritt der Umsetzung von zwei Äquivalenten Retinol mit einem Äquivalent Carbonyldiimidazol umfaßt.

7. Verfahren nach Anspruch 5 oder 6, **dadurch gekennzeichnet, daß** die Umsetzung in einem wasserfreien Medium und in einer inerten Atmosphäre durchgerührt wird.

8. Zusammensetzung, die in einem physiologisch akzeptablen Medium mindestens ein Retinolderivat nach einem der Ansprüche 1 bis 4 enthält.

9. Zusammensetzung nach Anspruch 8, **dadurch gekennzeichnet, daß** sie ferner mindestens einen Wirkstoff enthält, der unter den Depigmentierungsmitteln, Emollientien, Hydratisierungsmitteln, Wirkstoffen gegen Seborrhoe, Wirkstoffen gegen Akne, Wirkstoffen, die den Haarwuchs fördern, Keratolytika und/oder abschuppenden Wirkstoffen, reizlindernden oder beruhigenden Wirkstoffen und deren Gemischen ausgewählt ist.

10. Verwendung der Zusammensetzung nach Anspruch 8 oder 9 zur kosmetischen Behandlung der Haut, insbesondere gegen Akne und/oder altersbedingte oder aktinische Alterung.

11. Verwendung der Zusammensetzung nach Anspruch 8 oder 9 zur kosmetischen Behandlung der Kopfhaut, insbesondere zur Bekämpfung des fettigen Aussehens des Haares und Haarausfall.

12. Verwendung des Retinolderivats nach einem der Ansprüche 1 bis 4 zur Herstellung einer Zusammensetzung, die dazu vorgesehen ist, Akne und/oder altersbedingte oder aktinische Hautalterung zu bekämpfen.

13. Verwendung des Retinolderivats nach einem der Ansprüche 1 bis 4 zur Herstellung einer Zusammensetzung, die dazu vorgesehen ist, das fettige Aussehen der Haare und Haarausfall zu bekämpfen.

14. Verfahren zur kosmetischen Behandlung der Haut, das das Auftragen einer Zusammensetzung nach Anspruch 8 oder 9 auf die Haut umfaßt.
